# EUROPEAN PATENT SPECIFICATION

(11) **EP 3 113 677 B1**
(45) Date of publication and mention of the grant of the patent: **16.05.2018**
(21) Application number: 15759100.9
(22) Date of filing: 03.03.2015
(51) Int. Cl.: A61F 5/02

(54) **PELVIC BINDERS**
BECKENBINDEMITTEL
CEINTURES PELVIENNES

(30) Priority: 04.03.2014 US 201461947912 P
(43) Date of publication of application: 11.01.2017
(73) Proprietor: RevMedx, Inc., Wilsonville, OR 97020 (US)
(72) Inventor: STEINBAUGH, John, Wilsonville, OR 97020 (US)
(74) Representative: Hanna Moore + Curley
(86) International application number: PCT/US2015/018501
(87) International publication number: WO 2015/134506

(56) References cited:
- US-A- 5 503 620
- US-A- 6 165 147
- US-A1- 2004 039 321
- US-A1- 2011 034 845
- US-A1- 2011 289 688
- US-A1- 2012 253 251
- US-A1- 2013 333 706
- US-B1- 6 964 644

## Description

### Technical Field

The present disclosure relates generally to the field of injury support devices, and more particularly, to pelvic binders.

### Background

When a patient has suffered a pelvic injury, such as a fracture, securing the patient's pelvis until the patient reaches a medical facility can ease a patient's discomfort and reduce the likelihood of further damage. Existing support devices are typically formed of a cloth material, which may bunch and fold when a medical professional attempts to slide it under a patient on the ground, and which may tear in the presence of gravel or rocks. This bunching and resistance may prevent the medical professional from properly positioning the support device and may require the professional to lift or otherwise move the patient in a manner that may cause pain and further damage. Additionally, existing support devices provide little to no visual guidance on the proper tension to be applied in order to effectively secure the patient's pelvis without over tightening and causing harm or under tightening and failing to achieve the proper stabilization. US2011/0289688, on which the preamble of claim 1 is based, relates to a pelvic support and pelvic support application apparatus. US2012025325 relates to an adjustable brace apparatus. US5503620 describes a back support belt apparatus and method.

### Brief Description of the Drawings

Embodiments will be readily understood by the following detailed description in conjunction with the accompanying drawings. To facilitate this description, like reference numerals designate like structural elements. Embodiments are illustrated by way of example, and not by way of limitation, in the figures of the accompanying drawings.
FIG. 1 is a perspective view of a pelvic binder, in accordance with various embodiments.
FIG. 2 is a top view of an elongate member that may be included in the pelvic binder of FIG. 1, in accordance with various embodiments.
FIG. 3 is a perspective view of a second attachment member that may be included in the pelvic binder of FIG. 1, in accordance with various embodiments.
FIGS. 4A and 4B are perspective and side views, respectively, of a second attachment member that may be included in the pelvic binder of FIG. 1, in accordance with various embodiments.
FIG. 5 is a perspective view of a first attachment member that may be included in the pelvic binder of FIG. 1, in accordance with various embodiments.
FIG. 6 is a top view of a buckle that may be included in the pelvic binder of FIG. 1, in accordance with various embodiments.
FIGS. 7 and 8 depict labels that may be included in the pelvic binder of FIG. 1, in accordance with various embodiments.
FIG. 9 depicts sample instructions that may be included with the pelvic binder of FIG. 1, in accordance with various embodiments.
FIG. 10 depicts an embodiment of a pelvic binder being positioned around a wearer's body. FIG. 11 depicts an embodiment of a pelvic binder when a second attachment member is coupled with a first attachment member via a buckle.
FIG. 12 depicts an embodiment of a pelvic binder having been tensioned until a stop was reached.
FIG. 13 depicts an embodiment of a pelvic binder secured around the pelvis of a wearer and properly tensioned.
FIG. 14 depicts an embodiment of the pelvic binder folded into a flat package for storage.

### Detailed Description

Embodiments of pelvic binders and associated techniques are disclosed herein. In some embodiments, a pelvic binder may include an elongate member formed of a plastic or metal material, a first attachment member coupled proximate to a first end of the elongate member and having an elastic portion and a reference portion, and a second attachment member removably coupled to the elongate member. The pelvic binder may also include a target indicator positioned proximate to the first end of the elongate member so as to indicate that the elastic portion is under a predetermined, desired amount of tension when the reference portion is aligned with the target indicator.

The pelvic binders disclosed herein may provide visual guidance in obtaining the proper tension, thereby improving over existing support devices. As noted above, most such devices provide little to no visual feedback on the proper tensioning. Some existing devices may provide audible feedback (e.g., a "click" when the proper tension is achieved), but these small audible signals are easily lost in noisy environments, such as battlefields, traffic accidents, and other emergency situations.

In the following detailed description, reference is made to the accompanying drawings, which form a part hereof wherein like numerals designate like parts throughout, and in which is shown by way of illustration embodiments that may be practiced. It is to be understood that other embodiments may be utilized and structural or logical changes may be made without departing from the scope of the present disclosure. Therefore, the following detailed description is not to be taken in a limiting sense, and the scope of embodiments is defined by the appended claims and their equivalents.

Various operations may be described as multiple discrete actions or operations in turn, in a manner that is most helpful in understanding the claimed subject matter. However, the order of description should not be construed as to imply that these operations are necessarily order dependent. In particular, these operations may not be performed in the order of presentation. Operations described may be performed in a different order than the described embodiment. Various additional operations may be performed and/or described operations may be omitted in additional embodiments.

For the purposes of the present disclosure, the phrase "A and/or B" means (A), (B), or (A and B). For the purposes of the present disclosure, the phrase "A, B, and/or C" means (A), (B), (C), (A and B), (A and C), (B and C), or (A, B and C).

The description uses the phrases "in an embodiment," or "in embodiments," which may each refer to one or more of the same or different embodiments. Furthermore, the terms "comprising," "including," "having," and the like, as used with respect to embodiments of the present disclosure, are synonymous.

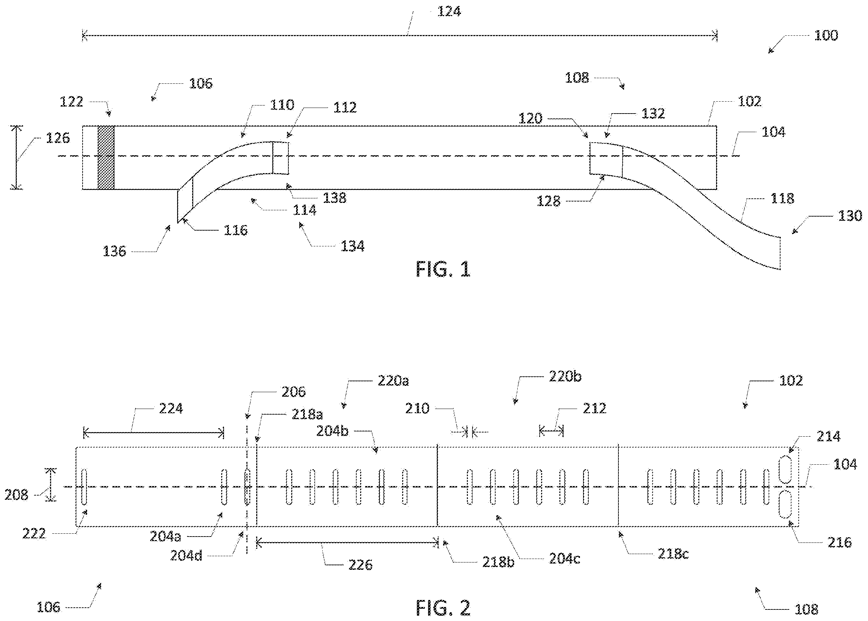

FIG. 1 is a perspective view of a pelvic binder 100, in accordance with various embodiments. The pelvic binder 100 may include an elongate member 102, a first attachment member 110 and a second attachment member 118. In use, the first attachment member 110 and the second attachment member 118 may couple to the elongate member 102 and to each other so that the pelvic binder 100 is arranged around a wearer's pelvis. When tension is applied to the second attachment member 118, the pelvic binder 100 may tighten around the wearer's pelvis until a desired predetermined amount of tension is reached and visually indicated. The pelvic binder 100 may substantially retain the predetermined tension while the wearer is transported to a medical facility for further treatment. A number of embodiments of the pelvic binder 100, and its various components, are discussed in detail below.

The elongate member 102 may have a longitudinal axis 104, and may have a longitudinal dimension 124 greater than approximately 101 cm (40 inches). In some embodiments, the longitudinal dimension 124 may be greater than approximately 127 cm (50 inches). The elongate member 102 may have a lateral dimension 126 greater than approximately 10.16 cm (4 inches). In some embodiments, the lateral dimension 126 may be greater than approximately 12.7 cm (5 inches). In some embodiments, the elongate member 102 may have a lateral dimension 126 equal to approximately 12.7 cm (5 inches) and a longitudinal dimension 124 approximately equal to 132.08 cm (52 inches). In some embodiments, the lateral dimension 126 and/or the longitudinal dimension 124 may be different for pelvic binders 100 intended for differently sized wearers (e.g., with smaller dimensions more appropriate for smaller wearers). The elongate member 102 may have a first end 106 and a second end 108 along the longitudinal axis 104.

The elongate member 102 may be formed of a plastic or metal material (e.g., a flexible plastic or metal material). For example, the elongate member 102 may be formed using one or more composite plastics, polypropylene, a malleable aluminum, a foam adhesive coating, and/or a fabric coating. In some embodiments, the material of the elongate member 102 may be sufficiently thin, rigid and smooth so as to be able to be positioned under a wearer's back or legs when the wearer is lying on the ground and slid under the wearer's hips for proper alignment with the trochanters, and may be sufficiently flexible in the longitudinal direction so as to be able to wrap around the wearer's pelvis. The elongate member 102 may not bunch when applied to a wearer in this manner. In some embodiments, the elongate member 102 may have a thickness in the range of approximately 0.508mm to approximately 0.127mm (20 thousandths of an inch to approximately 50 thousandths of an inch). Forming the elongate member 102 out of a plastic or other non-metallic material may advantageously allow the pelvic binder 100 to accompany the wearer into an x-ray or magnetic resonance imaging (MRI) chamber, thereby maintaining the stabilization of the pelvis during these diagnostics. In some embodiments, the elongate member 102 may be folded or separated (cut, torn, etc.) along scored or thinned sections in order to adjust the longitudinal dimension 124 and/or the lateral dimension 126. Various embodiments of the elongate member 102 are discussed below with reference to FIG. 2.

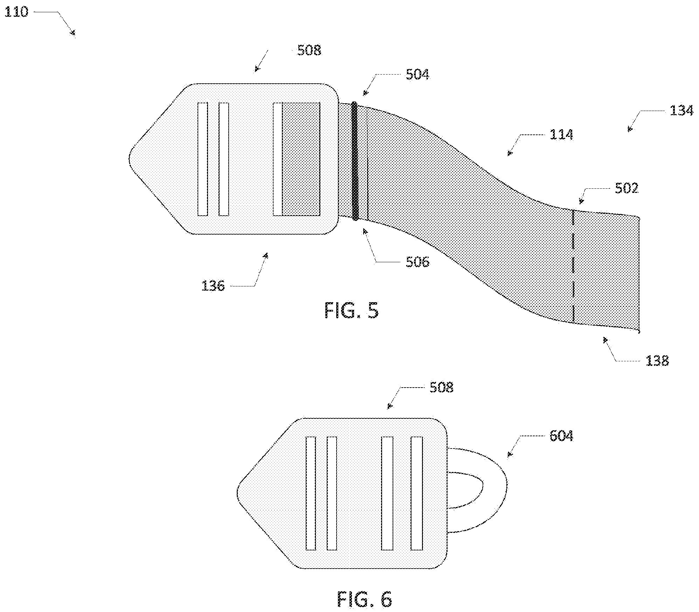

The first attachment member 110 may be coupled to the elongate member 102 at a first coupling point 112 via an attachment portion 138 proximate to a first end 134. The first attachment member 110 may include nylon or other webbing or strap material. For example, the first attachment member 110 may include a nylon strap that is 5.08cm (2 inches) wide. The attachment portion 138 of the first attachment member 110 may be configured for removable coupling between the first attachment member 110 and the elongate member 102, or for substantially permanent coupling. For example, in some embodiments, the elongate member 102 may include a hook or loop portion to which a complementary hook or loop portion of the attachment portion 138 may removably attach. In some embodiments, the elongate member 102 may include one or more male/female snaps to which one or more complementary snaps of the attachment portion 138 may removably attach. In some embodiments, the attachment portion 138 may be glued, sewn, fused or otherwise substantially permanently attached to the elongate member 102. Embodiments in which the first attachment member 110 is coupled to the elongate member 102 by fasteners or other mechanisms included wholly in the attachment portion 138 are discussed below with reference to FIG. 5.

The first attachment member 110 may include an elastic portion 114. The attachment portion 138 may be included in the elastic portion 114, or the elastic portion 114 may be coupled to the attachment portion 138 (e.g., by a stitched or fused seam). The elastic portion 114 may include any suitable stretchable material that may be dimensioned so that when the elastic portion 114 is stretched to a predetermined tension when securing the pelvic binder 100 to a wearer, the pelvic binder 100 provides a visual indicator that this tension has been achieved. The elastic portion 114 may include a woven elastic, rubber, a spring, rubber tubing, rubber strap, bungee cord, elastic polymer or any suitable elastic material. In some embodiments, the elastic portion 114 may include two or more layers of elastic material provided to increase the tension strength of the elastic portion 114 relative to only one layer of elastic material. For example, if one layer of elastic material of a given dimension is configured to provide 4.53kg (10 pounds) of tension, two layers of elastic material of the given dimension may provide 9.07 kg (20 pounds) of tension, and three layers of elastic material of the given dimension may provide 13.60kg (30 pounds) of tension. Consequently, multiple layers of an elastic material may be included in the elastic portion 114 to increase the amount of tension provided for a given length and/or width of the elastic portion 114.

The first attachment member 110 may include a reference portion 116. The reference portion 116 may be coupled with the elastic portion 114 (e.g., by gluing, sewing, or fusing). In some embodiments, the reference portion 116 may be disposed proximate to the second end 136 of the first attachment member 110. In some embodiments, the reference portion 116 may not be elastic, and may be substantially rigid such that any deformation of the first attachment member 110 when tension is applied may be attributed to deformation of the elastic portion 114. As discussed below, in some embodiments, alignment of the reference portion 116 with a target indicator on the elongate member 102 (such as the target indicator 122, discussed below) may signal proper tensioning of the pelvic binder 100. Various embodiments of the reference portion 116 are discussed below with reference to FIGS. 5 and 6.

The second attachment member 118 may be coupled to the elongate member 102 and a second coupling point 120 via an attachment portion 132. The second attachment member 118 may include nylon or other webbing or strap material. For example, the second attachment member 118 may include a nylon strap that is 5.08cm (2 inches) wide. The second coupling point 120 may be located between the second end 108 of the elongate member 102 and the first coupling point 112. In some embodiments, the elongate member 102 may provide multiple coupling point options for the second coupling point 102, and the second attachment member 118 may be removably coupled to the elongate member 102 at one of these coupling points. Various such embodiments are discussed below. The second attachment member 118 may include a first end 128 and a second end 130.

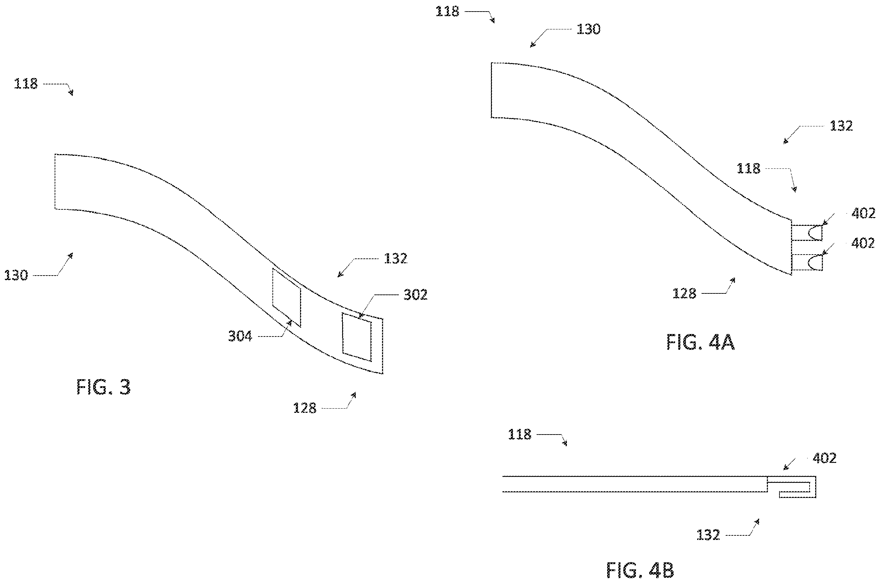

The attachment portion 132 of the second attachment member 118 may be configured for removable coupling between the second attachment member 118 and the elongate member 102, or for substantially permanent coupling. For example, in some embodiments, the elongate member 102 may include a hook or loop portion to which a complementary hook or loop portion of the attachment portion 132 may removably attach. In some embodiments, the elongate member 102 may include one or more male/female snaps to which one or more complementary snaps of the attachment portion 132 may removably attach. In some embodiments, the attachment portion 132 may be glued, sewn, fused or otherwise substantially permanently attached to the elongate member 102. Embodiments in which the second attachment member 118 is coupled to the elongate member 102 by fasteners or other mechanisms included wholly in the attachment portion 132 are discussed below with reference to FIGS. 3 and 4.

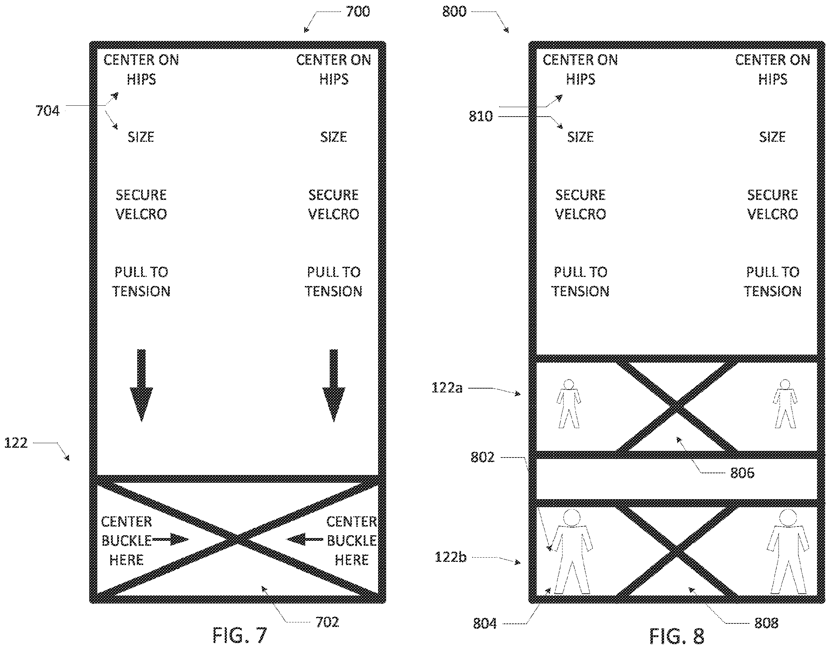

The pelvic binder 100 may also include a target indicator 122. The target indicator 122 may be configured such that, when the reference portion 116 is aligned with the target indicator 122, the elastic portion 114 of the first attachment member 110 is under a predetermined amount of tension. This amount of tension may be an amount appropriate for stabilizing a pelvic fracture or other injury without causing excessive discomfort or further harm to the wearer. In some embodiments, the target indicator 122 may be positioned proximate to the first end 106 of the elongate member 102. The target indicator 122 may be printed on or etched in the elongate member 102, or printed on a sticker attached to the elongate member 102. In some embodiments, the target indicator 122 may include notches, cutouts, colors, or other visual indicators to indicate the proper alignment of the reference portion 116. A number of embodiments of the target indicator 122 are discussed below with reference to FIGS. 7 and 8.

FIG. 2 is a top view of the elongate member 102, in accordance with various embodiments. Various embodiments of the elongate member 102 may include some, all, or none of the features of the elongate member 102 illustrated in and discussed below with reference to FIG. 2.

The elongate member 102 may include a plurality of elongate member sections 220. Only certain of the sections 220 (such as the sections 220a and 220b) are illustrated in FIG. 2 for clarity. The sections 220 may be separated by hinge portions 218 (such as the hinge portions 218a, 218b and 218c). The elongate member 102 may include one or more hinge portions 218 to divide the elongate member 102 into two or more sections 220. In some embodiments, a hinge portion 218 may include a lateral scoring, thinning or perforation of the elongate member 102 that may make it easier to laterally bend the elongate member 102 at the hinge portion 218 than at other portions of the elongate member 102. In some embodiments, the lateral scoring, thinning or perforation at a hinge portion 218 may enable a user to tear the elongate member 102 at the hinge portion 218 or more readily cut the elongate member 102 with a pair of scissors at the hinge portion 218 than at other portions. The ability to fold or tear the elongate member 102 at the hinge portions 218 may allow the length of the elongate member 102 to be decreased by folding one or more sections 220 on top of each other or tearing off one or more sections 220, thereby adjusting the circumference of the pelvic binder 100 to more appropriately fit differently sized wearers. In some embodiments, the elongate member 102 may be readily cut with a pair of scissors at any point. The elongate member 102 may include any desired number of hinge portions 218. For example, the elongate member 102 may include three hinge portions 218. In some embodiments, the elongate member 102 may include three hinge portions 218 disposed at a spacing 226 such that the elongate member 102 may fold in an accordion style into four 33.02cm (13-inch) sections (e.g., for storage). In some embodiments, the elongate member 102 may not include any hinge portions 218.

The elongate member 102 may include a plurality of slots 204. Only certain of the slots 204 (such as the slots 204a, 204b, 204c and 204d) are illustrated in FIG. 2 for clarity. Two or more of the slots 204 in the elongate member 102 may be substantially the same shape or may have substantially the same orientation. The elongate member 102 may include slots of different shapes and/or orientations.

In some embodiments, two or more slots 204 may be arranged along the longitudinal axis 104 of the elongate member 102. Some or all of the slots 204 may have longitudinal axes (such as the longitudinal axis 206) oriented substantially perpendicular to the longitudinal axis 104 of the elongate member 102. In some embodiments, a length 208 of a slot 204 may be selected to be slightly larger than a width of a strap that is to pass through the slot 204 (e.g., a strap of the first attachment member 110 or the second attachment member 118). For example, the length 208 of the slot 204 may be approximately 5.08cm (2 inches). In some embodiments, a width 210 of a slot 204 may be selected to be slightly larger than a thickness of a strap that is to pass through the slot 204 (e.g., a strap of the first attachment member 110 or the second attachment member 118). For example, the width 210 of the slot 204 may be approximately 0.635cm (1 quarter inch).

In some embodiments, the slots 204 may be arranged substantially regularly along the longitudinal axis 104. The arrangement of the slots 204 may be regular within different ones of the sections 220, as shown. In some embodiments, the spacing 212 between centers of adjacent slots 204 may be approximately 5.08cm (2 inches). The slots 204 in different ones of the sections 220 may be arranged so that if the elongate member 102 is folded at one or more of the hinge portions 218, the slots 204 in overlapping sections 220 may themselves overlap so as to allow the first attachment member 110 and/or the second attachment member 118 to pass through the slots 204 in multiple sections 220 of the elongate member 102. For example, if the elongate member is folded at the hinge portion 218b, the slot 204b may align with the slot 204c so that a strap or other element may pass through both the slot 204b and the slot 204c. Thus, if one or more sections 220 of the elongate member 102 are folded on top of each other, the first attachment member 110 and/or the second attachment member 118 may be secured to the overlapping region in the same manner as the first attachment member 110 and/or the second attachment member 118 may be attached to a single thickness of the elongate member 102. This may allow the elongate member 102 to be shortened in use without having extraneous material getting in the way of medical professionals or getting caught and jarring the wearer.

The elongate member 102 may include additional slots or other features to improve the ability of users to arrange the pelvic binder 100 on and around a wearer. For example, in some embodiments, the elongate member 102 may include a handle at the first end 106 and/or a handle at the second end 108. In some embodiments, a handle may take the form of one or more slots proximate to the first end 106 and/or the second end 108. For example, in FIG. 2, two laterally adjacent slots 214 and 216 are illustrated as disposed proximate to the second end 108. The laterally adjacent slots 214 and 216 may provide finger holes for a user to grasp when arranging the pelvic binder 100 around the body of a wearer. Handles included at the first end 106 and/or the second end 108 may take the form of such finger holes, or may be loops or other elements configured for ready grasping by a user. Handles may also be disposed along the length of the elongate member 102 in any desired location.

In some embodiments, the arrangement of slots 204 and other features may be different proximate to the first end 106 than to the second end 108. Different arrangements may be suitable, for example, when the coupling mechanism between the first attachment member 110 and the elongate member 102 is different from the coupling mechanism between the second attachment member 118 and the elongate member 102. For example, in some embodiments, the first attachment member 110 may be substantially permanently secured to the elongate member 102 by running the attachment portion 138 through the slots 204a and 204d and securing the attachment portion 138 to itself (e.g., by sewing, gluing or fusing). Because the attachment portion 138 may not be removable from the elongate member 102, a plurality of slots 204 proximate to the first end 106 (to serve as candidates for the first coupling point 112) may not be necessary or desired. In particular, since deformation of the elastic portion 114 of the first attachment member 110 is used to control and indicate the amount of tension applied by the pelvic binder 100 during use, having a fixed first coupling point 112 relative to the target indicator 122 may be useful for ensuring that the reference portion 116 will align with the target indicator 122 only when the elastic portion 114 has deformed to the predetermined desired amount.

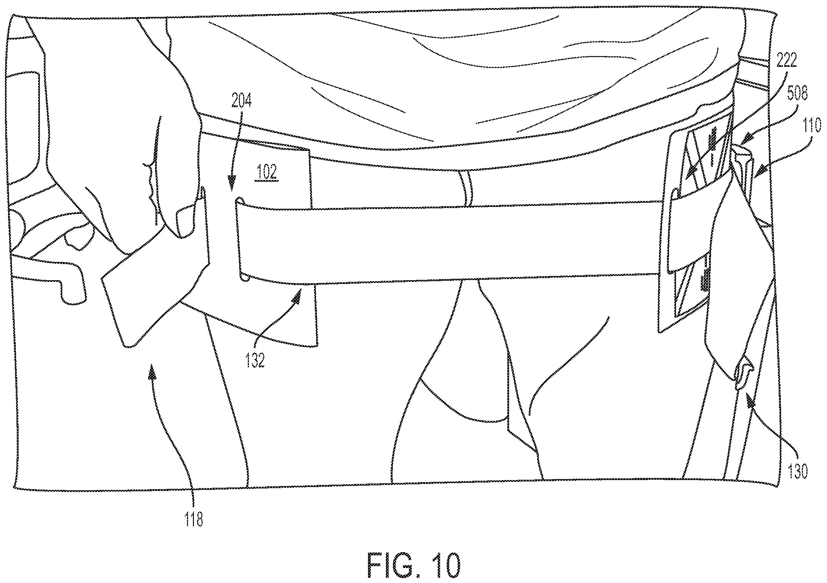

In some embodiments, the elongate member 102 may include a first end slot 222 disposed proximate to the first end 106. In use, the second attachment member 118 may be fed through the first end slot 222 before coupling with the first attachment member 110. The first end slot 222 may constrain the first attachment member 110 and the second attachment member 118 to a proper position around the wearer's pelvis by preventing the first attachment member 110 and the second attachment member 118 from sliding laterally. In some embodiments, the first end slot 222 may provide a "stop" for the reference portion 116 during tightening to prevent the pelvic binder 100 from being excessively tightened. Some such embodiments are discussed below (e.g., with reference to FIG. 10). In some embodiments, the elastic portion 114 and the first end slot 222 may be configured so that the tension of the pelvic binder 100 is approximately 18.14 kg (40 pounds) when the stop is reached. The center of the first end slot 222 may be spaced away from the center of the slot 204a by a distance 224 of at least approximately 15.24 cm (6 inches).

FIG. 3 is a perspective view of the second attachment member 118, in accordance with various embodiments. Various embodiments of the second attachment member 118 may include some, all, or none of the features of the second attachment member 118 illustrated in and discussed below with reference to FIG. 3.

The second attachment member 118 illustrated in FIG. 3 may include a first end 128, a second end 130 and the attachment portion 132. In some embodiments, the second attachment member 118 may include a strap, and the second end 130 may be configured to couple with the first attachment member 110. In some embodiments, the second end 130 may be a free end of the strap and may be configured to be inserted into and tightened around a buckle of the first attachment member 110 (e.g., as discussed below with reference to FIG. 5). In some embodiments, the second end 130 may include a male or female portion of a snap-lock buckle configured to mate with a complementary portion of the snap-lock buckle included in the first attachment member 110.

The attachment portion 132 may be configured to couple the second attachment member 118 to the elongate member 102. In some embodiments, the attachment portion 132 may include a fastener 302 and its complement 304 (e.g., a hook and loop portion and its complement, or a male/female snap and its complement), and the attachment portion 132 may be coupled to the elongate member 102 by running the attachment portion 132 through two slots 204 (or a loop or other portion of the elongate member 102) and then fastening the fastener 302 and its complement 304 around the slot or loop. In some embodiments, the attachment portion 132 may be run through two slots 204 (or a loop or other portion) of the elongate member 102 and then glued, sewn, fused or otherwise substantially permanently attached to itself around the slot or loop. Thus, in some embodiments, the slots 204, loop or other portion of the elongate member 102 may provide the second coupling point 120.

Other mechanisms may be used to couple the second attachment member 118 to the elongate member 102. FIGS. 4A and 4B depict perspective and side views, respectively, of the second attachment member 118, in accordance with various embodiments. Various embodiments of the second attachment member 118 may include some, all, or none of the features of the second attachment member 118 illustrated in and discussed below with reference to FIGS. 4A and 4B. The attachment portion 132 of the second attachment member 118 illustrated in FIG. 4A may include one or more hooks 402 disposed proximate to the first end 128. As shown in the side view of FIG. 4B, the hooks 402 may be substantially flat; and in some embodiments, the hooks 402 may be configured to be secured to a slot 204 of the elongate member 102 (FIG. 2). Since the second attachment member 118 may be under tension during use, the hooks 402 may remain coupled with the elongate member 102. In some embodiments, the hooks 402 may be plastic flat hooks. In some embodiments, the hooks 402 may squeeze the thickness of the material of the elongate member 102 when in use, thus providing additional resistance to slippage.

FIG. 5 is a perspective view of the first attachment member 110, in accordance with various embodiments. Various embodiments of the first attachment member 110 may include some, all, or none of the features of the first attachment member 110 illustrated in and discussed below with reference to FIG. 5.

The first attachment member 110 illustrated in FIG. 5 may include a first end 134 and a second end 136. The first end 134 may include an attachment portion 138 configured to couple the first attachment member 110 to the elongate member 102. In some embodiments, the attachment portion 138 may include a fastener and its complement (not shown, e.g., a hook and loop portion and its complement, or a male/female snap and its complement), and the attachment portion 138 may be coupled to the elongate member 102 by running the attachment portion 138 through two slots 204 (e.g., the slots 204a and 204d) and then fastening the fastener and its complement around the slots. In some embodiments, the attachment portion 138 may be run through two slots 204 (e.g., the slots 204a and 204d) of the elongate member 102, and then glued, sewn, fused or otherwise substantially permanently attached to itself around the slots (e.g., at a seam 502). Thus, in some embodiments, the slots 204 of the elongate member 102 may provide the first coupling point 112. In some embodiments, the first attachment member 110 may be substantially permanently secured to the elongate member 102.

An elastic portion 114 may be coupled between the attachment portion 138 and a buckle 508 disposed at the second end 136. In some embodiments, the elastic portion 114 may be coupled to a non-elastic portion 506 (e.g., by sewing, gluing or fusing) and the non-elastic portion 506 may be coupled to the buckle 508 (e.g., by sewing, gluing or fusing). In some embodiments, the non-elastic portion 506 may include a nylon webbing or other strap material (e.g., as discussed above with reference to the attachment portion 138). In some embodiments, the elastic portion 114 may be coupled to the buckle 508 (e.g., by sewing, gluing or fusing) without a non-elastic portion 506.

The first attachment member 110 may include a reference portion (e.g., the reference portion 116). In some embodiments, the buckle 508 may serve as the reference portion 116. In particular, the buckle 508 may be configured to receive the second end 130 of the second attachment member 118; as tension is applied to the second attachment member 118 to tighten the pelvic binder 100 around the wearer's pelvis, the buckle 508 may be pulled toward the first end 106 of the elongate member 102 and toward the target indicator 122. When the buckle 508 aligns with the target indicator 122, the desired tension has been achieved. In some embodiments, the first attachment member 110 may include an alignment marker 504 in addition to the buckle 508. The alignment marker 508 may be included in the non-elastic portion 506. Just as the buckle 508 may be pulled toward the first end 106 of the elongate member 102 as tension is applied to the second attachment member 118, the alignment marker 504 may be similarly pulled. When the alignment marker 504 aligns with the target indicator 122, the desired tension has been achieved. In some embodiments, the alignment marker 504 may include a grommet or other window in the elastic portion 114; when the grommet or window aligns with the target indicator 122, the desired tension has been achieved.

Although the buckle 508 depicted in FIG. 5 is configured to receive a free end of a strap of the second attachment member 118, any suitable buckle or other mechanism may be used to couple the first attachment member 110 and the second attachment member 118. In particular, any mechanism that allows tension to be applied to the pelvic binder 100 so as to stretch the elastic portion 114 to a desired length may be used. For example, in some embodiments, the buckle 508 may include a male or female portion of a snap-lock buckle, and may be configured to couple with a complementary portion disposed on the second attachment member 118. Once the snap-lock buckle is fashioned, a strap of the second attachment member 118 may be pulled through this complementary portion to tighten the pelvic binder 100 and pull the reference portion 116 toward the target indicator 122.

In some embodiments, the first attachment member 110 may include a handle or other element that the user may grasp when tensioning the pelvic binder 100 so as to provide a counterforce to prevent the wearer of the pelvic binder 100 from being pulled or jarred during the tensioning. For example, FIG. 6 illustrates a handle 604 extending from a buckle 508, in accordance with various embodiments. During use, the first attachment member 110 may be coupled with the second attachment member 118, and a user may grasp the handle 604 with one hand while grasping the second end 130 of the second attachment member 118 with the other hand. When the user pulls the second end 130 of the second attachment member 118 to tighten the pelvic binder 100, the user may apply a counterforce to the handle 604 to prevent the wearer from being rocked or pulled. In some embodiments, a handle may not extend from the buckle 508, but may extend from another portion of the first attachment member 110 (e.g., from the non-elastic portion 506).

The target indicator 122 may take any of a number of forms. For example, in some embodiments, the pelvic binder 100 may include two or more target indicators 122, each corresponding to a tension that is appropriate for a different size or age of person, and/or pelvic injury. FIGS. 7 and 8 depict labels 700 and 800, respectively, which may be included on the elongate member 102 and may include target indicators and other information. Various embodiments of labels may include some, all, or none of the features of the labels illustrated in and discussed below with reference to FIGS. 7 and 8.

The label 700 includes a target indicator 122 that includes a graphical "X" 702 to indicate the location of proper alignment of the reference portion 116. In particular, when the reference portion 116 is aligned with the center of the graphical "X" 702, the proper tension has been achieved. In some embodiments, a black graphical "X" on a white background may be advantageously visible even in low-light environments, and thus may facilitate the positioning of the reference portion 116 in such conditions. The label 700 may include textual instructions 704 that provide additional explanation regarding the proper alignment of the reference portion 116 (here, "center buckle here" and an arrow) and operation of the pelvic binder 100.

The label 800 includes two target indicators 122a and 122b. The target indicators 122a and 122b each include a graphical "X" (806 and 808, respectively) and a visual representation of a person (802 and 804, respectively). The visual representations included in the target indicators 122a and 122b are different, representing differently sized people. In the arrangement shown in the label 800, greater tension may be required to align the reference portion 116 (FIG. 1) with the target indicator 122b than to align the reference portion 116 with the target indicator 122b. Thus, with a larger person (e.g., an adult corresponding to the target indicator 122b), a greater tension may be suitable than with a smaller person (e.g., a child corresponding to the target indicator 122a). The label 800 may also include textual instructions 810 that provide additional explanation regarding the proper alignment of the reference portion 116 and operation of the pelvic binder 100.

In various embodiments, multiple target indicators 122 may be included in the pelvic binder 100 to correspond to different pelvic injuries. For example, some tensions may be suitable for fractures in certain parts of the pelvis, while other tensions may be suitable for fractures in other parts of the pelvis. The target indicators 122 may include graphical representations of the corresponding appropriate pelvic injuries.

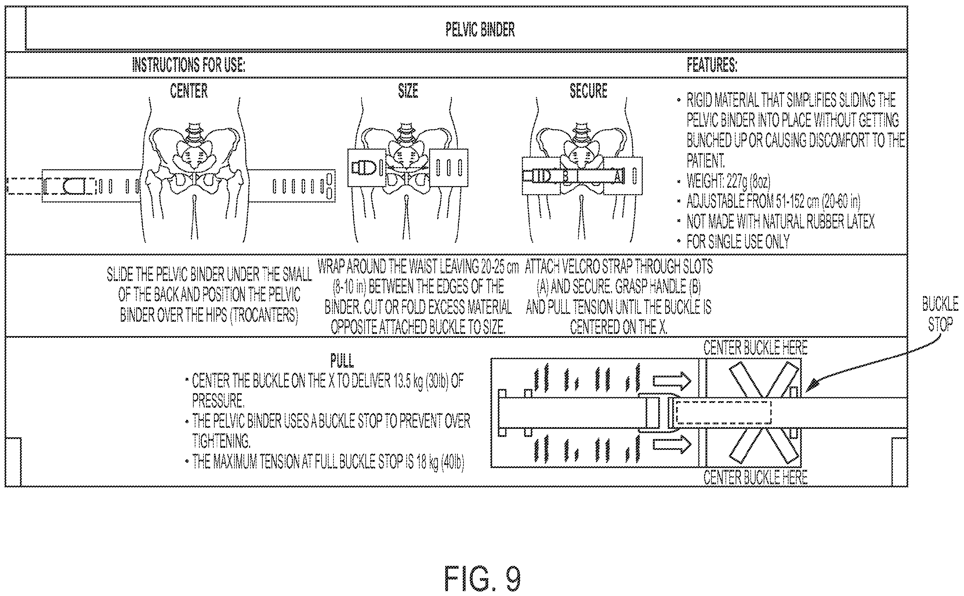

Instructions for the use of the pelvic binder 100 may be included in packaging along with the pelvic binder 100. FIG. 9 depicts sample instructions that may be included with the pelvic binder 100. The instructions of FIG. 9 describe the operation of certain embodiments of the pelvic binder 100 disclosed herein; however, these instructions may be readily modified to describe the operation of any of the pelvic binders disclosed herein. In some embodiments, different portions of the pelvic binder 100 may be color-coded, with these colors associated with printed instructions. For example, the first attachment member 110 may be a red color, the second attachment member 118 may be a blue color, and the printed instructions may indicate how the user is to manipulate the red and blue components. In some embodiments, different ends of the first attachment member 110 and/or the second attachment member 118 may have different colors to aid a user in coupling the attachment members to the elongate member 102 and to each other. For example, the first end 128 of the second attachment member 118 may be colored yellow, the second end 130 of the second attachment member 118 may be colored blue, and the accompanying instructions may indicate that the user is to secure the yellow portion to the elongate member 102 and pull on the blue portion to tighten.

FIG. 10 depicts an embodiment of the pelvic binder 100 being positioned around a wearer's body. As shown, the elongate member 102 may be positioned proximate to the wearer's trochanters. One of the sections of the elongate member 102 may be folded at a hinge portion and the attachment portion 132 of the second attachment member 118 may be threaded through overlapping sets of slots 204. Hook and loop material disposed on the second attachment member 118 may allow the first end of the second attachment member 118 to be secured to itself, thereby coupling the second attachment member 118 and the elongate member 102. The second end 130 of the second attachment member 118 may be run through the first end slot 222 and then through the buckle 508 included in the first attachment member 110. The second end 130 of the second attachment member 118 may be free.

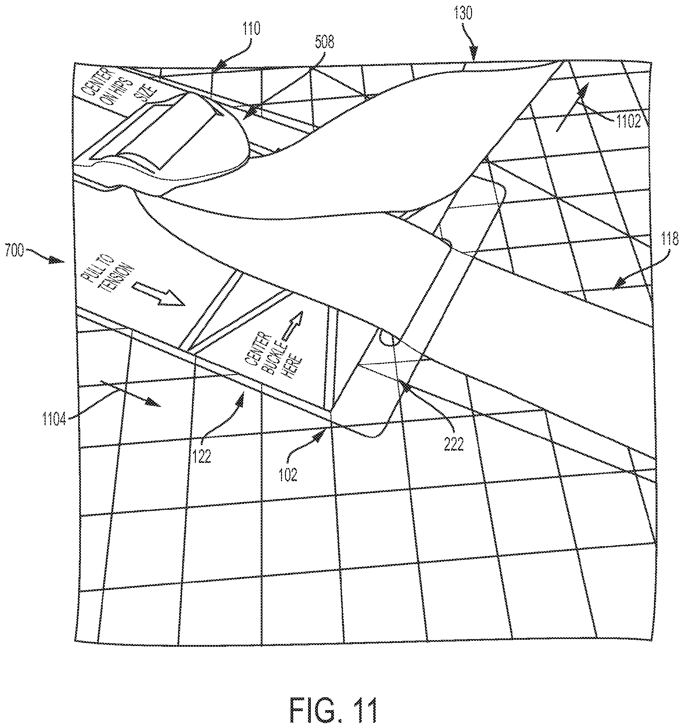

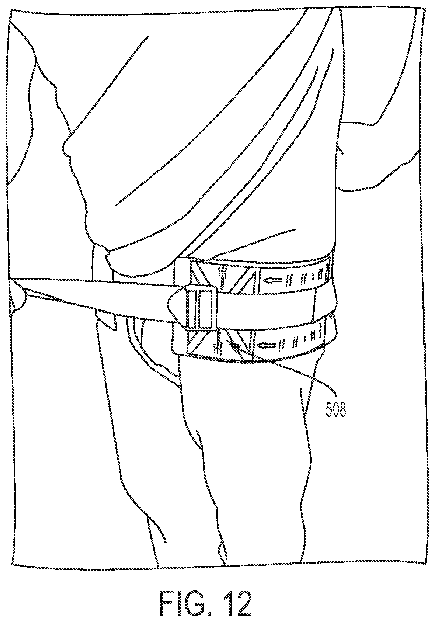

FIG. 11 depicts an embodiment of the pelvic binder 100 when the second attachment member 118 is coupled with the first attachment member 110 via the buckle 508 and the label 700 is included on the elongate member 102. In this embodiment, the buckle 508 may serve as the reference portion 116. When the second end 130 of the second attachment member 118 is pulled in the direction indicated by the arrow 1102, the buckle 508 may slide in the direction indicated by the arrow 1104. When the buckle 508 is aligned with the target indicator 122, the proper tension is achieved. If the second end 130 of the second attachment member 118 is pulled past the target indicator 122, further tightening will be prevented by the reference portion 116 (here, the buckle 508) running into the first end slot 222 of the elongate member 102. FIG. 12 depicts the buckle 508 having been pulled until the buckle 508 reached the first end slot 222.

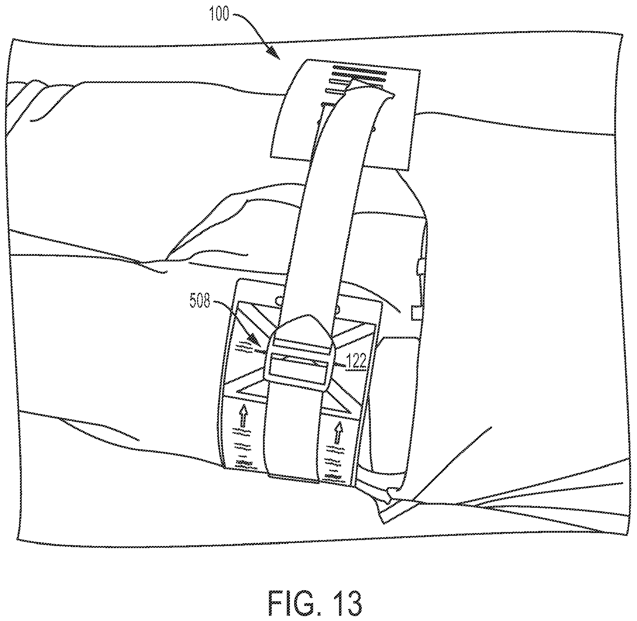

FIG. 13 depicts an embodiment of the pelvic binder 100 when the pelvic binder 100 is secured around the pelvis of a wearer and the reference portion 116 (here, the buckle 508) is properly aligned with the target indicator 122.

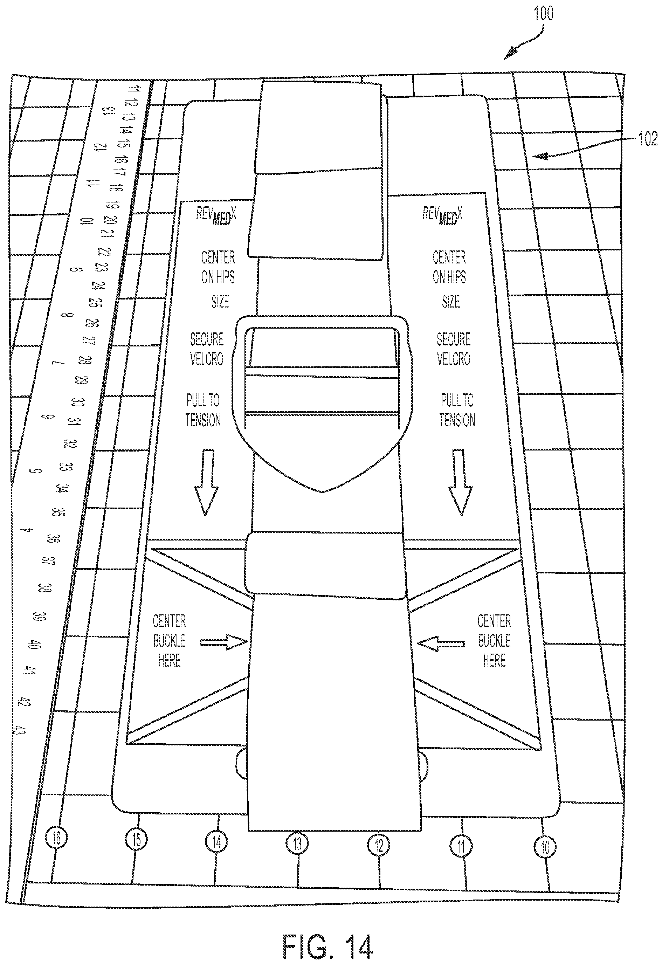

FIG. 14 depicts an embodiment of the pelvic binder 100 folded into a flat package for storage. In some embodiments, the elongate member 102 may be folded at the hinge portions 218 and the first attachment member 110 and the second attachment member 118 may be wrapped around the folded elongate member 102 to form a substantially flat package. This package may be vacuum sealed in a plastic or other impermeable wrapping. Instructions may be included within the wrapping or on the wrapping. In some embodiments, when the packaging containing the pelvic binder 100 is opened, the first attachment member 110 may already be coupled to the elongate member 102 (e.g., in a removable or substantially permanent manner). A user may then couple the second attachment member 118 at a desired position on the elongate member 102, and proceed with coupling the second attachment member 118 to the first attachment member 110 around the body of the wearer.

In some embodiments, the elongate member 102 may have one or both faces covered with a removable film prior to use. This film may be peeled back or perforated in the regions of the slots 204 so that a desired number or arrangement of slots 204 may be uncovered (e.g., the slots that may be used to attach the first attachment member 110 and/or the second attachment member 118). The remaining slots 204 may remain covered by the film. In embodiments in which this film covers the face of the elongate member 102 facing the wearer, the film may effectively "smooth over" the slots 204 in order to reduce the possibility of the formation of painful pressure points on the wearer's body arising from contact with the edges of the slots 204. Such pressure points may be a particular concern when the pelvic binder 100 is applied to the wearer for a significant period of time (e.g., 24 hours or more). In some embodiments in which a film is applied to the wearer-facing face of the elongate member 102, the attachment portion 138 of the first attachment member 110 and/or the attachment portion 132 of the second attachment member 118 may include a rigid portion to aid in piercing the film when securing the first attachment member 110 and/or the second attachment member 118 to the elongate member 102. In some embodiments the rigid portion may be a plastic backing sewn to a strap of the first attachment member 110 and/or the second attachment member 118. For example, the rigid portion may include a thermoplastic acrylic-polyvinyl chloride material (such as KYDEX®). In some embodiments, the materials included in the pelvic binder 100 may be used to perform other injury support functions in addition to or instead of pelvic binding. For example, in some embodiments, the elongate member 102 may be used as a splint when arranged along the length of a fractured leg or arm bone. If sufficiently flexible or malleable, the elongate member 102 may be folded longitudinally to double its thickness and thereby provide additional splint support. Since storage space is at a premium for emergency medical professionals, soldiers, hikers and other potential users of the pelvic binder 100, the ability of the pelvic binder 100 to perform multiple duties yields additional benefits.

In some embodiments, the elongate member 102 of the pelvic binder 100 may be formed from a fabric or textile, and may be provisioned with a first attachment member 110 and a second attachment member 118 in accordance with any of the embodiments discussed herein to provide proper tensioning when in use.

## Claims

1. A pelvic binder (100), comprising:
an elongate member (102) having a longitudinal axis (104), a first end (106) and a second end (108);
a first attachment member (110), coupled proximate to the first end of the elongate member (102) at a first coupling point (112);
a second attachment member (118), coupled to the elongate member (102) at a second coupling point (120) located between the second end of the elongate member (102) and the first coupling point (112); **characterized in that**
the first attachment member (110) comprises an elastic portion (114) and a reference portion (116), and
the pelvic binder (100) comprises a target indicator (122) positioned proximate to the first end of the elongate member (102) so as to indicate that the elastic portion (114) is under a predetermined amount of tension when the reference portion (116) is aligned with the target indicator (122).

2. The pelvic binder of claim 1, wherein the elongate member (102) includes a plurality of slots (204) distributed along the longitudinal axis (104) of the elongate member (102).

3. The pelvic binder of claim 2, wherein each slot (204) of the plurality of slots has a longitudinal axis oriented substantially perpendicular to the longitudinal axis (104) of the elongate member (102).

4. The pelvic binder of claim 2, wherein the elongate member includes one or more hinge portions (218).

5. The pelvic binder of claim 4, wherein:
a hinge portion (218) separates a first elongate member section (220) from a second elongate member section (220);
a first slot (204) is disposed in the first elongate member section (220a) and a second slot is disposed in the second elongate member section (220b); and
the first and second slots (204) are arranged such that, when the elongate member (102) is folded at the hinge portion and the first and second elongate member sections (220a, 220b) are overlaid, the first slot is aligned with the second slot.

6. The pelvic binder of claim 1, wherein the elongate member (102) includes one or more hinge portions (218), optionally wherein a hinge portion of the one or more hinge portions comprises a lateral scoring, thinning or perforation of a plastic or metal material.

7. The pelvic binder of claim 1, wherein the second attachment member (118) comprises a strap.

8. The pelvic binder of claim 7, wherein the strap has an attachment portion at a first end for coupling the second attachment member to the elongate member, and
a second end of the strap is configured to removably couple with the first attachment member, optionally wherein the attachment portion comprises a section of hook material and a section of loop material.

9. The pelvic binder of claim 1, wherein the first attachment member (110) comprises a strap having a first end secured to the elongate member and a second end secured to the elastic portion.

10. The pelvic binder of claim 1, wherein the second attachment member (118) comprises a strap, and the first attachment member (110) comprises a buckle configured to receive the strap, optionally wherein:
the elongate member (102) comprises a slot (204) proximate to the first end;
the slot (204) is configured to receive the strap; and
the slot (204) is positioned so as to prevent movement of the buckle (508) toward the first end of the elongate member when the buckle reaches the slot.

11. The pelvic binder of claim 1, wherein the reference portion (116) comprises a buckle (508) and the second attachment member (118) comprises a strap configured to be received by the buckle, optionally wherein applying tension to the strap when the strap is received by the buckle pulls the buckle toward the first end of the elongate member.

12. The pelvic binder of claim 1, comprising 2 or more target indicators (122) positioned at different distances from the first end of the elongate member (102).

13. The pelvic binder of claim 1, wherein the predetermined amount of tension is between 9 and 23kg (20 and 50 pounds).

14. A method of manufacturing a pelvic binder (100), comprising:
providing an elongate member (102) having a longitudinal axis (104), a first end and a second end;
providing a first attachment member (110), coupled proximate to the first end of the elongate member at a first coupling point, comprising an elastic portion and a reference portion;
providing a second attachment member (118), removably coupled to the elongate member at a second coupling point located between the second end of the elongate member and the first coupling point; and
providing a target indicator (122) positioned proximate to the first end of the elongate member so as to indicate that the elastic portion is under a predetermined amount of tension when the reference portion is aligned with the target indicator.

15. The method of claim 14, wherein providing the first attachment member (110) and providing the second attachment member (118) comprises
providing the first attachment member (110) coupled to the second attachment member (118), or
providing the target indicator (122) comprises causing the target indicator to be printed on the elongate member (102).

## Patentansprüche

1. Beckengurt (100), umfassend:
ein längliches Element (102), das eine Längsachse (104), ein erstes Ende (106) und ein zweites Ende (108) aufweist;
ein erstes Befestigungselement (110), das nahe dem ersten Ende des länglichen Elements (102) an einem ersten Kopplungspunkt (112) gekoppelt ist;
ein zweites Befestigungselement (118), das an einem zweiten Kopplungspunkt (120), der sich zwischen dem zweiten Ende des länglichen Elements (102) und dem ersten Kopplungspunkt (112) befindet, mit dem länglichen Element (102) gekoppelt ist; **dadurch gekennzeichnet, dass**
das erste Befestigungselement (110) einen elastischen Abschnitt (114) und einen Bezugsabschnitt (116) umfasst, und
der Beckengurt (100) einen Zielindikator (122) umfasst, der nahe dem ersten Ende des länglichen Elements (102) positioniert ist, um anzuzeigen, dass sich der elastische Abschnitt (114) unter einer vorbestimmten Menge an Spannung befindet, wenn der Bezugsabschnitt (116) auf den Zielindikator (122) ausgerichtet ist.

2. Beckengurt nach Anspruch 1, wobei das längliche Element (102) eine Mehrzahl von Schlitzen (204) umfasst, die entlang der Längsachse (104) des länglichen Elements (102) verteilt sind.

3. Beckengurt nach Anspruch 2, wobei jeder Schlitz (204) der Mehrzahl von Schlitzen eine Längsachse aufweist, die im Wesentlichen senkrecht zu der Längsachse (104) des länglichen Elements (102) ausgerichtet ist.

4. Beckengurt nach Anspruch 2, wobei das längliche Element einen oder mehrere Gelenkabschnitte (218) umfasst.

5. Beckengurt nach Anspruch 4, wobei:
ein Gelenkabschnitt (218) einen ersten Längselementabschnitt (220) von einem zweiten Längselementabschnitt (220) trennt;
ein erster Schlitz (204) in dem ersten Längselementabschnitt (220a) angeordnet ist und ein zweiter Schlitz in dem zweiten Längselementabschnitt (220b) angeordnet ist; und
der erste und der zweite Schlitz (204) derart angeordnet sind, dass, wenn das längliche Element (102) an dem Gelenkabschnitt gefaltet wird und der erste und der zweite Längselementabschnitt (220a, 220b) sich überlappen, der erste Schlitz auf den zweiten Schlitz ausgerichtet ist.

6. Beckengurt nach Anspruch 1, wobei das längliche Element (102) einen oder mehrere Gelenkabschnitte (218) umfasst, wobei optional ein Gelenkabschnitt des einen oder der mehreren Gelenkabschnitte eine seitliche Kerbung, Verdünnung oder Perforierung eines Kunststoff- oder Metallmaterials umfasst.

7. Beckengurt nach Anspruch 1, wobei das zweite Befestigungselement (118) einen Riemen umfasst.

8. Beckengurt nach Anspruch 7, wobei der Riemen an einem ersten Ende einen Befestigungsabschnitt zum Koppeln des zweiten Befestigungselements an das längliche Element aufweist, und
ein zweites Ende des Riemens dazu ausgelegt ist, lösbar mit dem ersten Befestigungselement zu koppeln, wobei optional der Befestigungsabschnitt einen Abschnitt mit Hakenmaterial und einen Abschnitt mit Schlaufenmaterial umfasst.

9. Beckengurt nach Anspruch 1, wobei das erste Befestigungselement (110) einen Riemen umfasst, der ein erstes Ende aufweist, das an dem länglichen Element fixiert ist, und ein zweites Ende, das an dem elastischen Abschnitt fixiert ist.

10. Beckengurt nach Anspruch 1, wobei das zweite Befestigungselement (118) einen Riemen umfasst und das erste Befestigungselement (110) eine Schnalle umfasst, die dazu ausgelegt ist, den Riemen aufzunehmen, wobei optional:
das längliche Element (102) einen Schlitz (204) nahe dem ersten Ende umfasst;
der Schlitz (204) dazu ausgelegt ist, den Riemen aufzunehmen; und
der Schlitz (204) derart positioniert ist, dass die Bewegung der Schnalle (508) in Richtung des ersten Endes des länglichen Elements verhindert wird, wenn die Schnalle den Schlitz erreicht.

11. Beckengurt nach Anspruch 1, wobei der Bezugsabschnitt (116) eine Schnalle (508) umfasst und das zweite Befestigungselement (118) einen Riemen umfasst, der dazu ausgelegt ist, von der Schnalle aufgenommen zu werden, wobei optional das Aufbringen von Spannung auf den Riemen, wenn der Riemen von der Schnalle aufgenommen wird, die Schnalle in Richtung des ersten Endes des länglichen Elements zieht.

12. Beckengurt nach Anspruch 1, der 2 oder mehrere Zielindikatoren (122) umfasst, die in verschiedenen Abständen von dem ersten Ende des länglichen Elements (102) positioniert sind.

13. Beckengurt nach Anspruch 1, wobei die vorbestimmte Menge an Spannung zwischen 9 und 23 kg liegt.

14. Verfahren zur Herstellung eines Beckengurts (100), umfassend:
Bereitstellen eines länglichen Elements (102), das eine Längsachse (104), ein erstes Ende (106) und ein zweites Ende (108) aufweist;
Bereitstellen eines ersten Befestigungselements (110), das nahe dem ersten Ende des länglichen Elements an einem ersten Kopplungspunkt gekoppelt ist, und das einen elastischen Abschnitt und einen Bezugsabschnitt umfasst;
Bereitstellen eines zweiten Befestigungselements (118), das an einem zweiten Kopplungspunkt, der sich zwischen dem zweiten Ende des länglichen Elements und dem ersten Kopplungspunkt befindet, mit dem länglichen Element lösbar gekoppelt ist; und
Bereitstellen eines Zielindikators (122), der nahe dem ersten Ende des länglichen Elements positioniert ist, um anzuzeigen, dass sich der elastische Abschnitt unter einer vorbestimmten Menge an Spannung befindet, wenn der Bezugsabschnitt auf den Zielindikator ausgerichtet ist.

15. Verfahren nach Anspruch 14, wobei das Bereitstellen des ersten Befestigungselements (110) und das Bereitstellen des zweiten Befestigungselements (118) umfasst:
Bereitstellen des ersten Befestigungselements (110), das mit dem zweiten Befestigungselement (118) gekoppelt ist, oder
das Bereitstellen des Zielindikators (122) das Bewirken, dass der Zielindikator auf das längliche Element (102) gedruckt wird, umfasst.

## Revendications

1. Ceinture pelvienne (100), comprenant :
un élément allongé (102) ayant un axe longitudinal (104), une première extrémité (106) et une seconde extrémité (108) ;
un premier élément de fixation (110), couplé de manière proximale à la première extrémité de l'élément allongé (102) au niveau d'un premier point de couplage (112) ;
un second élément de fixation (118), couplé à l'élément allongé (102) au niveau d'un second point de couplage (120) situé entre la seconde extrémité de l'élément allongé (102) et le premier point de couplage (112) ; **caractérisée en ce que**
le premier élément de fixation (110) comprend une partie élastique (114) et une partie de référence (116), et
la ceinture pelvienne (100) comprend un indicateur de cible (122) positionné à proximité de la première extrémité de l'élément allongé (102) de façon à indiquer que la partie élastique (114) est sous une quantité prédéterminée de tension lorsque la partie de référence (116) est alignée avec l'indicateur de cible (122).

2. Ceinture pelvienne selon la revendication 1, dans laquelle l'élément allongé (102) comprend une pluralité de fentes (204) réparties le long de l'axe longitudinal (104) de l'élément allongé (102).

3. Ceinture pelvienne selon la revendication 2, dans laquelle chaque fente (204) de la pluralité de fentes a un axe longitudinal orienté d'une manière sensiblement perpendiculaire à l'axe longitudinal (104) de l'élément allongé (102).

4. Ceinture pelvienne selon la revendication 2, dans laquelle l'élément allongé comprend une ou plusieurs parties charnières (218).

5. Ceinture pelvienne selon la revendication 4, dans laquelle :
une partie charnière (218) sépare une première section d'élément allongé (220) vis-à-vis d'une seconde section d'élément allongé (220) ;
une première fente (204) est disposée dans la première section d'élément allongé (220a) et une seconde fente est disposée dans la seconde section d'élément allongé (220b) ; et
les première et seconde fentes (204) sont agencées de telle sorte que, lorsque l'élément allongé (102) est plié au niveau de la partie charnière et que les première et seconde sections d'élément allongé (220a, 220b) sont superposées, la première fente est alignée avec la seconde fente.

6. Ceinture pelvienne selon la revendication 1, dans laquelle l'élément allongé (102) comprend une ou plusieurs parties charnières (218), une partie charnière parmi la ou des parties charnières comprenant facultativement un traçage latéral, un amincissement latéral ou une perforation latérale d'une matière plastique ou métallique.

7. Ceinture pelvienne selon la revendication 1, dans laquelle le second élément de fixation (118) comprend une sangle.

8. Ceinture pelvienne selon la revendication 7, dans laquelle la sangle a une partie de fixation au niveau d'une première extrémité pour coupler le second élément de fixation à l'élément allongé, et
une seconde extrémité de la sangle est configurée pour être couplée de manière amovible au premier élément de fixation, la partie de fixation comprenant facultativement une section de matériau formant crochet et une section de matériau formant boucle.

9. Ceinture pelvienne selon la revendication 1, dans laquelle le premier élément de fixation (110) comprend une sangle ayant une première extrémité fixée à l'élément allongé et une seconde extrémité fixée à la partie élastique.

10. Ceinture pelvienne selon la revendication 1, dans laquelle le second élément de fixation (118) comprend une sangle, et le premier élément de fixation (110) comprend une boucle configurée pour recevoir la sangle, facultativement :
l'élément allongé (102) comprenant une fente (204) à proximité de la première extrémité ;
la fente (204) étant configurée pour recevoir la sangle ; et
la fente (204) étant positionnée de façon à empêcher un déplacement de la boucle (508) vers la première extrémité de l'élément allongé lorsque la boucle atteint la fente.

11. Ceinture pelvienne selon la revendication 1, dans laquelle la partie de référence (116) comprend une boucle (508) et le second élément de fixation (118) comprend une sangle configurée pour être reçue par la boucle, appliquer une tension sur la sangle lorsque la sangle est reçue par la boucle tirant facultativement la boucle vers la première extrémité de l'élément allongé.

12. Ceinture pelvienne selon la revendication 1, comprenant au moins 2 indicateurs de cible (122) positionnés à différentes distances de la première extrémité de l'élément allongé (102).

13. Ceinture pelvienne selon la revendication 1, dans laquelle la quantité prédéterminée de tension est entre 9 et 23 kg (20 et 50 livres).

14. Procédé de fabrication d'une ceinture pelvienne (100), comprenant :
disposer un élément allongé (102) ayant un axe longitudinal (104), une première extrémité et une seconde extrémité ;
disposer un premier élément de fixation (110), couplé de manière proximale à la première extrémité de l'élément allongé au niveau d'un premier point de couplage, comprenant une partie élastique et une partie de référence ;
disposer un second élément de fixation (118), couplé de manière amovible à l'élément allongé au niveau d'un second point de couplage situé entre la seconde extrémité de l'élément allongé et le premier point de couplage ; et
disposer un indicateur de cible (122) positionné à proximité de la première extrémité de l'élément allongé de façon à indiquer que la partie élastique est sous une quantité prédéterminée de tension lorsque la partie de référence est alignée avec l'indicateur de cible.

15. Procédé selon la revendication 14, dans lequel disposer le premier élément de fixation (110) et disposer le second élément de fixation (118) comprend
disposer le premier élément de fixation (110) couplé au second élément de fixation (118), ou
disposer l'indicateur de cible (122) comprend amener l'indicateur de cible à être imprimé sur l'élément allongé (102).
